Europäisches Patentamt

European Patent Office

Office européen des brevets

(11) Publication number: **0 252 564 B1**

## EUROPEAN PATENT SPECIFICATION

(45) Date of publication of patent specification: **14.04.93**

(21) Application number: **87201275.2**

(22) Date of filing: **01.07.87**

(51) Int. Cl.5: **C12P 13/02**, C12N 1/20, //C12N9/78,(C12N1/20, C12R1:01)

(54) **Process for the preparation of difluorobenzamide.**

(30) Priority: **02.07.86 GB 8616160**

(43) Date of publication of application:
**13.01.88 Bulletin 88/02**

(45) Publication of the grant of the patent:
**14.04.93 Bulletin 93/15**

(84) Designated Contracting States:
**AT BE CH DE ES FR GB GR IT LI LU NL SE**

(56) References cited:
EP-A- 0 133 927
EP-A- 0 178 106
FR-A- 2 294 999

CHEMICAL ABSTRACTS, vol. 96, no. 21, 24th
May 1982, page 701, abstract no. 181159j,
Columbus, Ohio, US; & JP-A-82 02 273
(ISHIHARA SANGYO KAISHA LTD) 07-01-1982

(73) Proprietor: **SHELL INTERNATIONALE RESEARCH MAATSCHAPPIJ B.V.**
**Carel van Bylandtlaan 30**
**NL-2596 HR Den Haag(NL)**

Proprietor: **GIST-BROCADES N.V.**
**Wateringseweg 1**
**NL-2611 XT Delft(NL)**

(72) Inventor: **Clifford, Kenneth Henry**
**14 Arundel Avenue**
**Sittingbourne Kent ME10 4RG(GB)**
Inventor: **Pryce, Robert John**
**3 Canterbury Road**
**Faversham Kent ME13 8JU(GB)**
Inventor: **Geary, Philip John**
**5 Old School Court Egerton**
**Ashford Kent TN27 9DW(GB)**

**Description**

This invention relates to a process for the preparation of 2,6-difluorobenzamide.

EP-A-133 927 (Nitto) discloses the conversion of a nitrile compound to the corresponding amide by the action of certain suitable microorganisms. These microorganisms are generally improved in their activity by irradiation with light. Specific types of microorganism disclosed as suitable are gram-positive bacteria of the genera:

    (a) Corynebacterium;

    (b) Nocardia;

    (c) Bacillus;

    (d) Bacteridium;

    (e) Micrococcus; and

    (f) Brevibacterium.

The prime utility disclosed is production of acrylamide from acrylonitrile and nicotinic acid from cyanopyridine. Additional conversions specifically disclosed are conversions of acetonitrile to acetamide, methacrylonitrile to methacrylamide, valeronitrile to valeramide, benzonitrile to benzamide, propionitrile to propionamide, n-butyronitrile to n-butyramide, malononitrile to malonamide, succinonitrile to succinamide, fumaronitrile to fumaramide, chloroacetonitrile to chloroacetamide and $\beta$-hydroxypropionitrile to $\beta$-hydroxypropionamide.

D.B. Harper, "Microbial Metabolism of Aromatic Nitriles", Biochem. J. (1977) 165, 309-319 describes the metabolism of benzonitrile (to benzoic acid) by Nocardia Sp. (rhodochrous group) NCIB 11216 and the isolation and properties of its nitrilase enzyme. Table 4 on Page 317 discloses the relative rates of hydrolysis by the enzyme of various substituted benzonitriles. It appears that the rate is very much affected by steric hindrance afforded by a substituent in the 2-position. Thus the rate for 2-fluorobenzonitrile is less than 30% of that for benzonitrile, and the rates for 2-chlorobenzonitrile and 2-methylbenzonitrile are substantially zero, whereas the rates for 4-fluorobenzonitrile, 4-chlorobenzonitrile and 4-methylbenzonitrile are significantly greater than that for benzonitrile. It may reasonably be predicted therefore that a 2,6-disubstituted benzonitrile, being even more sterically hindered, would be wholly unsusceptible to the enzymatic hydrolysis. Indeed, Harper found that 2,6-dichlorobenzonitrile (the herbicide Dichlobenil) was not attacked even at high concentrations. Even 3,5-dibromobenzonitrile (the herbicide Bromoxynil) remained unattacked at high concentrations of the enzyme, notwithstanding the fact that the rate of hydrolysis for 3-bromobenzonitrile was more than 70% greater than for benzonitrile. Harper also records that Verloop, Residue Rev. 43 (1972) 55-103 found that mono-orthosubstitution decreases rate of alkaline hydrolysis of aromatic nitriles but di-orthosubstitution completely inhibits the process.

It has now surprisingly been discovered that 2,6-difluorobenzonitrile can be hydrolysed microbially. Furthermore, a novel microorganism has surprisingly been found which is capable of hydrolysing 2,6-difluorobenzonitrile at a faster rate than benzonitrile per se.

According to the present invention therefore there is a provided a process for the preparation of 2,6-difluorobenzamide which comprises subjecting 2,6-difluorobenzonitrile to the action of a suitable hydrolysing bacterium of the genus Rhodococcus or nitrilase extract therefrom.

Conveniently the microorganism per se is used in the process, but if desired nitrilase extract obtained from the microorganism in known manner may be used instead.

A preferred example of a species of bacterium of the genus Rhodococcus has been obtained as a soil isolate from the sludge treatment plant at Shell Haven Refinery, Stanford-le-Hope, Essex, England and has been deposited at the National Collection of Industrial Bacteria (NCIB), Torrey Research Station, 135 Abbey Road, Aberdeen AB9 8DG, Scotland, on 13th March 1986 under the provisions of the Budapest Treaty on the International Recognition of the Deposit of Microorganisms for the Purposes of Patent Procedure and has been assigned accession number NCIB 12218. This microorganism is herein referred to as "Rhodococcus Sp. NCIB 12218".

This novel microorganism, Rhodococcus Sp. NCIB 12218, is, as will be described in detail hereinafter, capable of hydrolysing 2,6-diflurobenzonitrile at a faster rate than it hydrolyses benzonitrile per se.

Accordingly in the process of the invention the microorganism is preferably Rhodococcus Sp. NCIB 12218 or a mutant thereof having the same properties.

The invention also comprises Rhodococcus Sp. NCIB 12218 and mutants thereof having the same properties.

Mutants of Rhodococcus Sp. NCIB 12218 may be generated, isolated and selected by known procedures. Thus mutation may be by chemical means, e.g. using N-methyl-N'-nitro-N-nitro-soguanidine, or by physical means employing ultraviolet radiation.

EP 0 252 564 B1

2,6-Difluorobenzamide is a known intermediate for the preparation of known insecticidal compounds, e.g. diflubenzuron (see UK Patent Specification No. 1,324,293). Other processes using 2,6-difluorobenzamide as an intermediate for insecticidally active compounds include those described in UK Patent Specification No. 1,460,419 and EP-A-161 019.

Microorganisms for use in the process of the invention may be grown on a suitable known growth medium containing an assimilable carbon source (e.g. glucose, lactose or sucrose), an assimilable nitrogen source (e.g. ammonium sulphate, ammonium nitrate or ammonium chloride), a source of organic trace nutrients (e.g. yeast extract, malt extract, peptone, or meat extract) and inorganic trace nutrients (e.g. phosphate, magnesium, potassium, calcium, manganese, zinc and/or iron salts).

Growth is generally effected at a temperature in the range 20 to 37°C and a pH in the range 5 to 9, with agitation (e.g. by shaking or stirring) in the presence of oxygen or air. Cells may be harvested in known manner and if desired kept on agar slopes, frozen or lyophilised before use in the process of the invention.

The process of the invention may conveniently be effected at a temperature in the range 0 to 37°C, preferably 20 to 30°C, at a pH in the range 6 to 9, preferably 7 to 8, and preferably with irradiation by light.

Rhodococcus Sp. NCIB 12218 has been characterised and identified by the NCIB as follows:

Tests were at 30°C and growth was on Oxoid CM3 Nutrient Agar unless otherwise stated.

Cell Morphology

After growth for 5 hours at 30°C on Oxoid CM1 Nutrient broth + 0.75%w agar, by phase contrast at x 630 magnification cells are small parallel-sided slightly bent or curved rods, with some clustering of cells.
Gram positive
Spores -
Motility -

Colonial Morphology

After 3 days growth, colonies are round, regular, entire, smooth, opaque, low convex, very pale pink and approximately 1mm in diameter.

Growth on Glucose Peptone Water Sugars

37°C ± trace
45°C -
Catalase +
Oxidase, Kovacs -
O-F glucose Oxidative
"O-F glucose" was performed using the oxidation-fermentation medium of Haywad and Hodgkiss, J.Gen. Microbiol. 26 (1961) 133-140, supplemented with 1%w filter-sterilised D-glucose. A tube sample was inoculated with Rhodococcus Sp. NCIB 12218 and incubated for 14 days.

Rhodococcus Sp. NCIB 12218 can conveniently be stored on nutrient agar slopes at 4°C, or cells may be isolated in buffer medium and stored at low temperatures, e.g. -15°C. It has been found possible to store cells at -15°C for a month without loss of activity.

The invention will be further understood from the following Examples.

EXAMPLE 1

Growth medium was made up from the following constituents:
2.0 g $K_2HPO_4$
0.2 g $MgSO_4.7H_2O$
2.5 mg $FeSO_4.7H_2O$
12.5 mg $CaCl_2.2H_2O$
2.5 mg $MnSO_4.3H_2O$
1.0 g $(NH_4)_2SO_4$
10 g Glucose
5 g Peptone
3 g Yeast extract

3

3 g Malt extract

These constituents were dissolved in 900 ml water, pH was adjusted to 7.2 by addition of hydrochloric acid and the solution was made up to 1 litre by addition of water.

50 ml of the above growth medium in a 250 ml conical flask was inoculated with Rhodococcus Sp. NCIB 12218 and incubated at 30°C on a shaker for 48 hours.

Cells were harvested by centrifugation (at 20,000 rpm for 15 minutes on a "Sorvall" (trade mark) centrifuge), were washed with a 50mM phosphate buffer of pH 8, recentrifuged and were resuspended in 2.5 ml of the buffer. A small sample of the suspension was removed and used to determine the dry weight of the cells therein.

A quantity of the suspension calculated to contain 0.5 mg dry weight of Rhodococcus Sp. NCIB 12218 cells was diluted to 1 ml with the above buffer. The suspension was maintained at 25°C and was irradiated for 5 minutes by means of a 60 W tungsten lamp at a distance of 25 cm before addition of 1 mg of 2,6-difluorobenzonitrile (DFBN). Irradiation was continued and the mixture was maintained at 25°C for 15 minutes after the addition of DFBN. A sample was then withdrawn and analysed by gas chromatography (gc) for DFBN and 2,6-difluorobenzamide (DFBAM) to determine the extent of reaction.

The above procedure was repeated for comparative purposes using benzonitrile and 2,6-dichlorobenzonitrile in place of the DFBN. Results are given in Table I following.

## Table 1

| Substrate | Product | Productivity |
|---|---|---|
| 2,6-difluoro-benzonitrile | 2,6-difluorobenzamide | 25.5g/g cells/hour (0.178 mol/g cells/hour) |
| benzonitrile | benzamide | 15.6g/g cells/hour (0.146 mol/g cells/hour) |
| 2,6-dichloro-benzonitrile | 2,6-dichlorobenzamide | 0.053g/g cells/hour ($3 \times 10^{-4}$ mol/g cells/hour) |

## EXAMPLE 2

400 ml of the growth of Example 1 was inoculated with Rhodococcus Sp. NCIB 12218 and incubated at 30°C for 48 hours.

The resulting cells were harvested and washed as in Example 1 and were suspended (440 mg dry weight) in 2 litres of the phosphate buffer of Example 1.

The cell suspension was stirred (magnetic stirrer) in a glass vessel at 25°C and irradiated for 5 minutes by means of three 60W tungsten lamps at a distance of 25 cm. 75 g DFBN was added, and the mixture was kept at 25°C, under irradiation, for 16 hours, at which stage analysis (gc) showed 98.9% conversion of DFBN to DFBAM.

DFBAM crystallised out of solution as it was formed. This crystalline precipitate was recovered directly and recrystallised from ethyl acetate to yield 50.3g DFBAM. After cooling and evaporation of the reaction mixture, a further 16 g DFBAM was isolated.

## EXAMPLE 3

50μl of a suspension of cells of Rhodococcus Sp. NCIB 12218 in phosphate buffer, was prepared and irradiated as in Example 2, and then added to a solution of 5μl of DFBN in 1 ml n-heptane. Irradiation and stirring were continued at room temperature. Crystals were deposited. After 1½ hours, a further 10μl of DFBN were added. After 18 hrs, the crystals were filtered off. Analysis showed virtually complete conversion of DFBN to DFBAM.

EXAMPLE 4

A suspension of cells of Rhodococcus Sp. NCIB 12218 in phosphate buffer was prepared as in Examples 1 and 2. 3.2 ml of this suspension was stirred (magnetic stirrer) in a glass vessel at 25°C and irradiated for 5 minutes as described in Example 2. n-Heptane was added to give a volume of 50ml. Irradiation and stirring continued at a temperature of 28°C, and 3.24g of DFBN was added. GLC analysis showed that 92% of the DFBN was converted to DFBAM after 5 hours.

**Claims**

1. A process for the preparation of 2,6-difluorobenzamide which comprises subjecting 2,6-difluorobenzonitrile to the action of a suitable hydrolysing bacterium of the genus Rhodococcus or nitrilase extract therefrom.

2. A process according to Claim 1 wherein the microorganism is Rhodococcus Sp. NCIB 12218 or a mutant thereof, having the same properties.

3. Rhodococcus Sp. NCIB 12218 or a mutant thereof having the same properties.

**Patentansprüche**

1. Verfahren zur Herstellung von 2,6-Difluorbenzamid, welches ein Einwirkenlassen eines geeigneten hydrolysierenden Bakteriums der Gattung Rhodococcus oder eines daraus erhaltenen Nitrilaseextraktes auf 2,6-Difluorbenzonitril umfaßt.

2. Verfahren nach Anspruch 1, worin der Mikroorganismus Rhodococcus Sp.NCIB 12218 oder eine Mutante hievon mit den gleichen Eigenschaften ist.

3. Rhodococcus Sp. NCIB 12218 oder eine Mutante hievon mit den gleichen Eigenschaften.

**Revendications**

1. Procédé de préparation de 2,6-difluorobenzamide, qui comprend le fait de soumettre du 2,6-difluorobenzonitrile à l'action d'une bactérie hydrolysante convenable du genre Rhodococcus ou à un extrait nitrilase de celle-ci.

2. Procédé selon la revendication 1, dans lequel le microorganisme est Rhodococcus sp. NCIB 12218, ou un mutant de celui-ci possédant les mêmes propriétés.

3. Rhodococcus sp. NCIB 12218, ou mutant de celui-ci possédant les mêmes propriétés.